# EUROPEAN PATENT APPLICATION

(11) **EP 2 359 885 A1**
(43) Date of publication of application: **24.08.2011**
(21) Application number: 09833540.9
(22) Date of filing: 09.03.2009
(51) Int. Cl.: A61M 5/158

(54) **STRUCTURE OF MICRO-NEEDLE WITH CHANNEL THEREINSIDE AND MANUFACTURING METHOD THEREOF**

(30) Priority: 18.12.2008 KR 20080129332
(71) Applicant: Miti Systems Inc., Daejeon 305-500 (KR)
(72) Inventor: LEE, Seung Seob, Daejeon 305-701 (KR); SUL, Boo Joon, Daejeon 305-701 (KR); HAN, Man Hee, Daejeon 305-701 (KR)
(74) Representative: Lacey, Dean Andrew
(86) International application number: PCT/KR2009/001146
(87) International publication number: WO 2010/071262

(57) **Abstract**

The present invention relates to a microneedle which is configured to have a vertical groove formed along a side thereof so as to serve as a fluid passageway and in which an upper portion of an outer edge of each of a pair of projections forming both sidewalls of the vertical groove is formed to be narrower than an outer edge of the body defining a bottom of the vertical groove and to be shorter than an upper portion of the body, a manufacturing method thereof, and an out-of-plane microneedle sheet to which the microneedle is applied. The microneedle can be provided which allows active ingredients or blood to effectively flow into or out of the human body through a space between the surface of a microneedle and a punctured skin texture or through the vertical groove.

## Description

### Technical Field

The present invention relates to a microneedle configured to have a fluid passageway formed along a side thereof and a method for manufacturing the same, and more particularly, to an in-plane microneedle having a fluid passageway formed along the side thereof, a manufacturing method therefor, and an in-plane and out-of-plane microneedle array to which the microneedle is applied and has a fluid passageway formed along the side thereof.

### Background of the Related Art

Recently, a method of delivering a skin care ingredient or drug into the human body by penetration thereof through the skin texture has an advantage in that it enables a sustainable drug delivery, is painless, and is simple and convenient in use, but also has a disadvantage in that since the stratum corneum which is an outermost layer of the epidermis of the skin and is 10-60 µm in depth inhibits the outflow of internal body substances and the penetration of external substances into the human body, transdermal absorption of active ingredients is extremely low. Particularly, if the active ingredients are hydrophilic or have a large molecular weight, the transdermal absorption thereof further decreases.

Thus, conventionally, a needle has been used which has a diameter measured in millimeter units (mm) and a length measured in centimeter units (cm) so as to be adapted to extract blood from the skin of the human body or inject a liquid medicine into the human body. Such a needle stimulates a plurality of pain spots widely distributed in the skin, which gives a considerable pain to a subject in use.

In order to address and solve the above problem, a microneedle has been developed which has a diameter of several tens to several hundreds of micrometers (µm) and a length of several hundreds to several thousands of micrometers (µm). Since this microneedle is relatively small in diameter and length as compared to the conventional needles, the number of pain spots stimulated is reduced, thereby resulting in significant alleviation of a pain given to the subject.

Since the use of one microneedle decreases drug delivery efficiency or greatly reduces the amount of extractable internal body substances, the microneedles are configured in an array in which a plurality of microneedles is arranged according to a predetermined arrangement pattern as shown in FIGs, 1 and 2. The microneedle array is divided into an in-plane microneedle array and an out-of-plane microneedle array depending on whether the arrangement of each microneedle is two-dimensional or three-dimensional.

In the meantime, there have been developed microneedle structures which are diversely modified so as to enhance percutaneous delivery efficiency of a drug or the like using such a microneedle array.

U.S. Pat. Nos. 3,964,482 and 6,256,533 disclose a hollow microneedle having a fluid passageway or channel formed therein so as to allow a drug to be transferred into the human body therethrough. However, since such a hollow microneedle has a diameter of several tens to several hundreds of micrometers and requires that it should have a fluid channel of a smaller diameter formed therein, it can be fabricated only by a highly precise work. Furthermore, this type of microneedle has a shortcoming that since a core used for forming the channel is relatively long as compared to the diameter of the microneedle, it can be deformed due to a force generated by the flow of a fluid or a pressure applied to a mold, which may occur during the manufacturing process.

U.S. Pat. No. 6,881,203 discloses a microneedle array including a plurality of microneedles each having a fluid channel formed in the outer surface of each microneedle and being in fluid communication with an optional conduit structure formed on the substrate surface along each row of microneedles. The above U.S. patent document teaches a method of fabricating a mold (out-of-plane microneedle array) in such a fashion as to engrave a substrate in the shape of three-dimensional microneedles to form cavities in the shape of the desired microneedles by using various processes such as laser ablation, photolithography, chemical etching, ion beam etching, etc. However, according to the above method, since the engravings such as the cavities must be formed as many as the number of microneedles to be installed on the microneedle array as well as a three-dimensional engraving must be performed, it is not easy to obtain various structures of more sophisticated microneedles.

Korean Patent No. 682534 as a prior art invented by the present inventors discloses a method for manufacturing a sophisticated and low-priced out-of-plane microneedle array which comprises performing photolithography and electroplating processes on a planar base to fabricate an in-plane microneedle array, and then vertically arranging the in-plane microneedle array on a planar substrate to fabricate the out-of-plane microneedle array (see FIGs. 3 and 4). Furthermore, the above Korean Patent document teaches a method for forming a vertical groove along a side of each microneedle in a longitudinal direction of the microneedle in order to facilitate the injection of a drug into the human body or the extraction of internal body substances. Specifically, the above Korean Patent document suggests a method of using a base 100 having a plurality of trenches 103 formed thereon by a variety of etching methods, a method of forming trenches 103 on the base 100 before the electroplating is performed, and a method of directly forming the vertical groove along the side of each microneedle using laser machining or electrical discharge machining (EDM).

However, since the microneedle formed with the vertical groove fabricated by the conventional methods has a low precision, it requires an inconvenient and difficult process in which the outer appearance of the microneedle is surface-finished sophisticatedly after a primary fabrication of the microneedle.

In the meantime, intracutaneous absorption rate of the active ingredients by the microneedle is determined depending on how fast the active ingredients from the outside of the human body are transferred to a skin depth where a diffusion rate thereof is high, and how fast the active ingredients are diffused into the human body. The microneedle formed with the vertical groove is intended to improving the active ingredient-transferring effect in the former. One method for improving the active ingredient-diffusing effect in the latter is to increase the area where the active ingredients are in contact with the skin. In the microneedle formed with the vertical groove, an increase in the width of the groove results in an increase in the area where the active ingredients are in contact with the skin. However, since the skin texture is high in elasticity, the skin serves to obstruct or clog the vertical groove, which results in a decrease in the speed at which the active ingredients are transferred to a skin depth where a diffusion rate thereof is high.

### SUMMARY OF THE DISCLOSURE

Therefore, the present invention has been made in view of the above-mentioned problems occurring in the prior art, and it is an object of the present invention to provide a microneedle which is configured to have a vertical groove formed along a side thereof and allows active ingredients or blood to effectively flow into or out of the human body through a space between the surface of a microneedle and a punctured skin texture or through the vertical groove.

Another object of the present invention is to provide a method of manufacturing the microneedle in a simple and economical manner through a consistent process.

Still another object of the present invention is to provide an in-plane microneedle array to which the microneedle is applied.

Yet another object of the present invention is to provide an out-of-plane microneedle array to which the microneedle is applied.

A further object of the present invention is to provide an out-of-plane microneedle array in which a sheet has a through-recess formed thereon so as to store and transport a drug and the through-recess and the vertical groove fluidically communicate with each other and to which the microneedle in which is applied.

In order to accomplish the above objects, the present invention provides a microneedle configured to have a vertical groove serving as a fluid passageway formed along a side thereof, a method for manufacturing the microneedle, and an out-of-plane microneedle array to which the microneedle is applied.

Terminology herein is merely used to describe specific embodiments of the invention, but is not intended to limit the invention. The singular forms "a", "an" and "the" include plural referents unless the context clearly dictates otherwise.

Hereinafter, in the present invention, a distal end of a microneedle is defined as a "tip". A bottom portion of the microneedle is defined as a "base". Sidewalls defining the vertical groove are defined as "projections". A portion which extends beneath the tip in such a fashion as to define a bottom of the vertical groove and abut against the rear surface of the projections is defined as a "body". Also, the sides of the projections and the body are defined as an "outer edges". In addition, the terms "upper portion" and "lower portion" of each microneedle as defined herein refer to a tip portion and a base portion which are in a relative positional relationship, respectively. The terms "upper surface" and "lower surface" of each microneedle as defined herein refer to a top surface of the projections defining the vertical groove and a bottom surface of the body opposite to the projections, respectively.

**Structure of microneedle**

The present invention is directed to a microneedle which is configured to have a vertical groove formed along a side thereof so as to serve as a fluid passageway and in which an upper portion of an outer edge of each of a pair of projections forming both sidewalls of the vertical groove is formed to be narrower than an outer edge of the body defining a bottom of the vertical groove and to be shorter than an upper portion of the body.

In the present invention, preferably, the pair of projections and the body become gradually narrower as they go toward the top, i.e., the tip of the microneedle.

**Method for manufacturing the microneedle**

Also, the present invention is directed to a method for manufacturing a microneedle which is configured to have a vertical groove formed along a side thereof so as to serve as a fluid passageway. The method comprises the steps of (a) forming a light-shielding pattern having the shape of a planar cross-section of the microneedle on a light-transmitting substrate; (b) coating the light-transmitting substrate with a photoresist, masking the photoresist in a light-shielding pattern corresponding to the vertical groove, and exposing the masked photoresist to light to thereby define projections for the vertical groove; (c) coating the light-transmitting substrate with a photoresist, exposing and developing the photoresist from the bottom surface of the light-transmitting substrate to thereby form a mold for the microneedle formed with the vertical groove; (d) depositing an electroplating seed layer on the top surface of the mold; and (e) removing a plating layer formed by performing a plating process on the electroplating seed layer.

In the present invention, each process of "exposing" the photoresist may mean the entire process ranging from the heat treatment to the development after the exposure, and may mean a process of performing the heat treatment after the exposure and performing the development in the last step. That is, the "exposing" of the photoresist in the above step (b) may include only the heat treatment after the exposure, and may include both the heat treatment and the development after the exposure. Whether the developments are to be performed each time the exposures are performed or are to be performed simultaneously lastly after the exposures can be determined arbitrarily. It will be appreciated by those skilled in the art that whatever alternative is selected, the result is the same, and hence it is not limited in the present invention. But in case where the development of the photoresist is performed in each step, the number of processes increases and concavo-convexes or corrugations may occur on the surface of substrate, which results in a possibility that a photoresist to be coated later will not be irregularly distributed on the substrate. The "exposure" which will be referred hereinafter also has the same meaning.

In the step (b) of the method for manufacturing a microneedle of the present invention, the light-shielding pattern corresponding to the vertical groove is configured in such a fashion that a slit corresponding to the vertical groove is formed in a tip of the microneedle, and the width and length thereof are smaller by a predetermined dimension than those of the light-shielding pattern having the planar cross-section shape of the microneedle in the step (a). In other words, the light-shielding pattern corresponding to the vertical groove is preferably positioned within the light-shielding pattern having the planar cross-section shape of the microneedle. Likewise, the light-shielding pattern corresponding to the vertical groove is sized and positioned to be superposed within the light-shielding pattern having the planar cross-section shape of the microneedle so as to prevent the tip of the vertical groove and the tip of the microneedle from being superposed on each other so that the tip of the microneedle can be fabricated in a clearly finished state.

In addition, in the present invention, the light-shielding pattern having the planar cross-section shape of the microneedle and the light-shielding pattern corresponding to the vertical groove preferably become narrower as they go toward the top.

In the present invention, the light-shielding pattern may have the shape of the planar cross-section of one microneedle or the shape of the planar cross-section of a plurality of microneedles which are arranged in parallel with each other. It is, of course, natural that in case of the latter, the light-shielding pattern corresponding to the vertical groove is configured such that a plurality of vertical grooves is arranged in parallel with each other to correspond to the plurality of microneedles in case of the former.

Moreover, in the present invention, the exposing of the photoresist in the step (c) is preferably performed in an inclined manner so that the tip of the microneedle is sharpened angularly.

Besides, the method for manufacturing the microneedle of the present invention may further include the steps of: (f) fabricating a negative mold using the removed plating layer as an original form; and (e) obtaining a molded product made of a polymer material using the negative mold.

Accordingly, a number of microneedles and microneedle sheets can be economically manufactured based on a small quantity of metallic form.

**Microneedle sheet to which the microneedle is applied**

Also, the present invention is directed to an out-of-plane microneedle sheet (array) to which the above-constructed microneedle is applied.

Preferably, the microneedle sheet includes a through-recess formed therein so as to store or transport a drug, the through-recess being adapted to fluidically communicate with the vertical groove of the microneedle. To this end, in the microneedle manufacturing process as described above, the lower portion of the light-shielding pattern corresponding to the vertical groove is preferably extended to be connected to the base of the microneedle. By doing so, active ingredients which will exist in the opposite side or the through-recess of the microneedle array can efficiently penetrate into the skin texture through the vertical groove of the microneedle.

Since a microneedle sheet or array to which a typical microneedle is applied and a method for manufacturing the same have been described in detail in the prior art, the detailed description thereof will be omitted to avoid redundancy.

According to the present invention, a microneedle can be provided which is configured to have a vertical groove formed along a side thereof and allows active ingredients or blood to effectively flow into or out of the human body through a space between the surface of a microneedle and a punctured skin texture or through the vertical groove.

Also, the microneedle formed along a side thereof with a micro-vertical groove, which serves as a fluid passageway, can be manufactured in a simple and economical manner through the exposure, development and plating processes only.

In addition, since the microneedle is fabricated in a state where the tip thereof has been clearly finished, a process of finishing the manufactured product can be omitted.

Moreover, in the case of employing the in-plane and out-of-plane microneedle array manufactured by using the microneedle formed with the vertical groove according the present invention, since the micro-vertical groove penetrating through the stratum corneum is formed by the microneedle, even a drug having a large molecular weight such as insulin, hormone formulation and the like can be transdermally delivered effectively. Further, when the drug penetrates into only the epidermal layer having no nerve fibers, it can be very easily painlessly delivered into the human body.

### BRIEF DESCRIPTION OF THE DRAWINGS

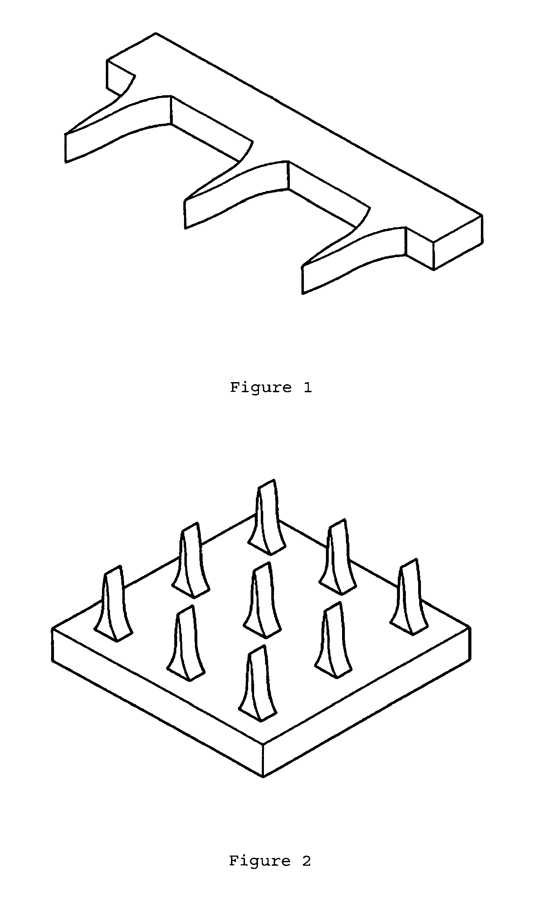

FIG. 1 is a perspective view illustrating an example of an in-plane microneedle array;

FIG. 2 is a perspective view illustrating an example of an out-of-plane microneedle array;

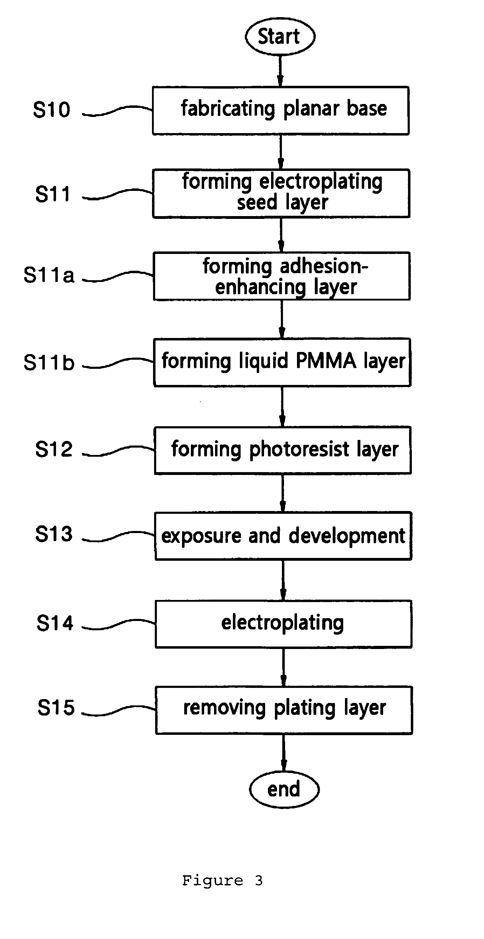

FIG. 3 is a flowchart illustrating a process of manufacturing an in-plane microneedle array according to the prior art;

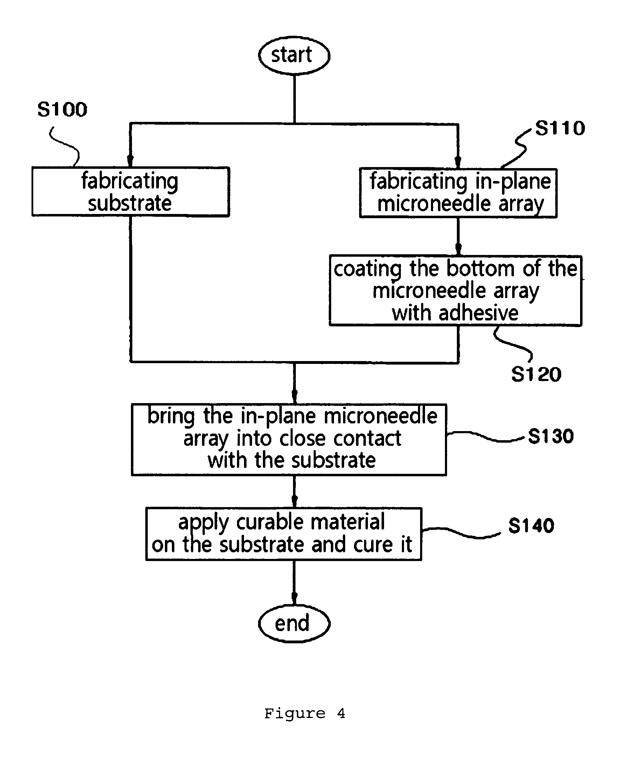

FIG. 4 is a flowchart illustrating a process of manufacturing an out-of-plane microneedle array according to the prior art;

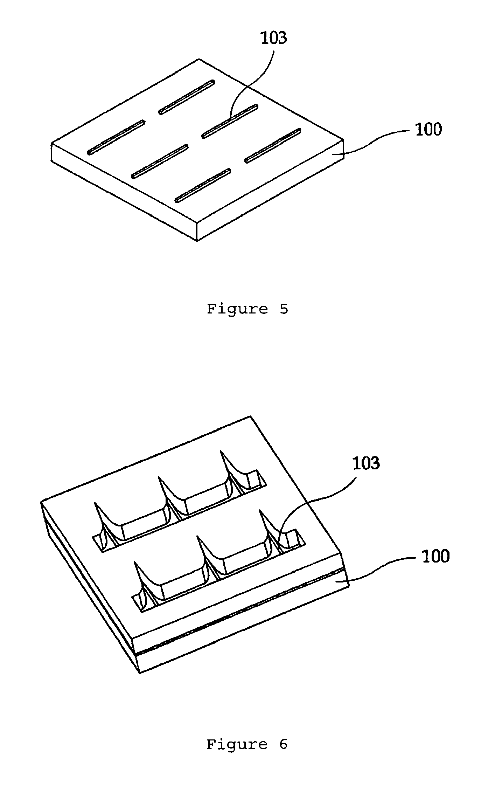

FIGs. 5 and 6 are perspective views illustrating a base having trenches formed thereon according to the prior art;

FIGs. 7 and 8 are perspective views illustrating an example of a microneedle according to the present invention and an example of a microneedle according to the prior art;

FIGs. 9 and 10 are conceptual views illustrating an example of a process for fabricating a mold for the microneedle formed with a vertical groove 1 according to the present invention;

FIG. 11 is a transverse cross-sectional view illustrating another example of a process for manufacturing the microneedle formed with a vertical groove 1 according to the present invention;

FIGs. 12 and 13 are a conceptual view illustrating the case where the pattern corresponding to the vertical groove 1 protrudes outwardly from the pattern of the tip 11 of the microneedle and a photograph of the microneedle manufactured as a result thereof in the microneedle manufacturing process according to the present invention;

FIGs. 14 and 15 are a conceptual view illustrating the case where the pattern corresponding to the vertical groove 1 is positioned within the pattern of the tip 11 of the microneedle and a photograph of the microneedle manufactured as a result thereof in the microneedle manufacturing process according to the present invention; and

FIG. 16 is a photograph illustrating an example of the manufacturing of an out-of-plane microneedle sheet 20 to which the microneedle of the present invention is applied.

### DETAILED DESCRIPTION OF THE DISCLOSURE

The present invention will be hereinafter described in detail in connection with the preferred embodiments with reference to the accompanying drawings. However, these embodiments of the present invention are merely illustrative of easy explanation on the contents and scope of the technical spirit of the present invention, but the technical scope of the present invention is not limited or modified thereby. Also, it will be understood by those skilled in the art that various modifications and variations can be made to the present invention without departing from the spirit and scope of the appended claims based on the illustrative embodiments.

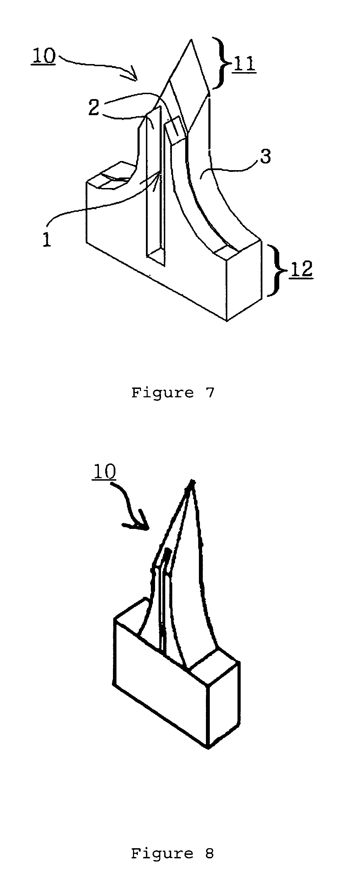

FIGs. 7 and 8 are perspective views illustrating an example of a microneedle according to the present invention and an example of a microneedle according to the prior art.

Referring to FIGs. 7 and 8, the microneedle according to the prior art is constructed such that a vertical groove 1 is simply formed at a side of an integral body 3. Such an integral microneedle has an effect that active ingredients from the outside of the human body are rapidly transferred to a skin depth. However, if the width of the groove increases so as to increase the area where the active ingredients are in close contact with the skin, the skin texture having a high elasticity is partially pushed forcedly into the vertical groove, so that it serves to obstruct or clog the vertical groove. Thus, although the conventional microneedle formed with the vertical groove 1 along a side thereof is higher in penetration speed of external active ingredients into the human body than a microneedle formed without the vertical groove 1 along a side thereof, it still have an inefficient aspect.

On the other hand, the microneedle according to the present invention has a structure that seems to define the vertical groove 1 by combination of three parts consisting of a body 3 and a pair of projections 2 in terms of an outer appearance. The pair of projections 2 and the body 1 are preferably get gradually narrower as they go toward the top, i.e., a tip 11 of the microneedle. In this case, which an upper portion of an outer edge of each of the pair of projections 2 is formed to be narrower than an outer edge of the body 3 and to be shorter than an upper portion of the body 3. That is, the microneedle of the present invention has a structure in which a pair of projections 2 seems to be disposed inside a top surface of the body 3. Thus, a slight free space is formed between the outer edges of the body 3 and the projections 2.

The microneedle of the present invention has the above structure so that when the microneedle to applied to the skin, the active ingredients transferred along the vertical groove reaches the free space formed between the outer edges of the body 3 and the projections 2 adjacent to the tip 11 side. In this case, since the free space increase the area in close contact with the skin texture, it can enhance a diffusion efficiency in which the active ingredients diffuse into the human body.

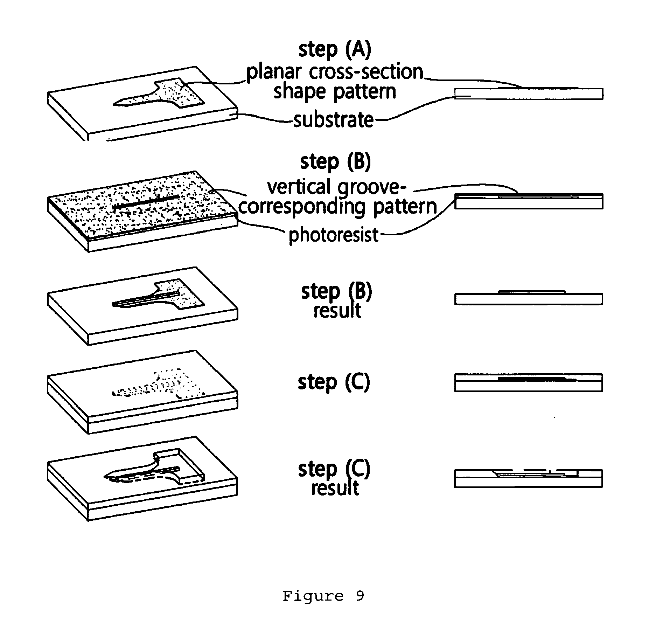

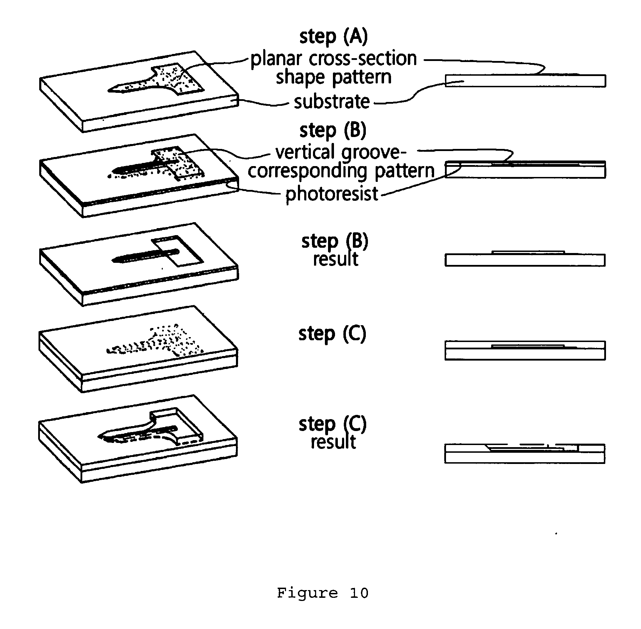

FIGs. 9 and 10 are conceptual views illustrating an example of a process for fabricating a mold for the microneedle formed with a vertical groove 1 according to the present invention.

In the mold fabricating processes of FIGs. 9 and 10, the remaining steps are the same except a difference in the structure of a light-shielding pattern corresponding to the vertical groove 1. In FIGs. 9 and 10, the figures on the left side are perspective views of the mold for the microneedle in the mold fabricating process, and the figures on the right side are longitudinal cross-sectional views of the mold for the microneedle in the mold fabricating process.
In the step (A), first, a light-shielding pattern having the shape of a planar cross-section of the microneedle is formed on a light-transmitting substrate. Subsequently, in the step (B), a photoresist is coated to a thickness corresponding to a depth of the vertical groove 1 on the light-transmitting substrate, and the photoresist is exposed to light using a mask having a light-shielding pattern corresponding to the vertical groove 1 to thereby define projections 2 for the vertical groove 1.

In the step (C), a photoresist is coated to a thickness the same as that of the microneedle on the light-transmitting substrate formed with the projections for the vertical groove 1, and then the photoresist is exposed to light and developed from the bottom surface of the light-transmitting substrate. Through this process, a mold for the microneedle formed with the vertical groove can be obtained.

Thereafter, although not shown, an electroplating seed layer is deposited on the top surface of the obtained mold (step (D)), and then a plating layer formed is removed by performing a plating process on the electroplating seed layer (step (E)) to thereby obtain the microneedle formed with the vertical groove 1.

A finishing process may be additionally provided in which the removed plating layer is subjected to grinding, lapping and polishing, if necessary.

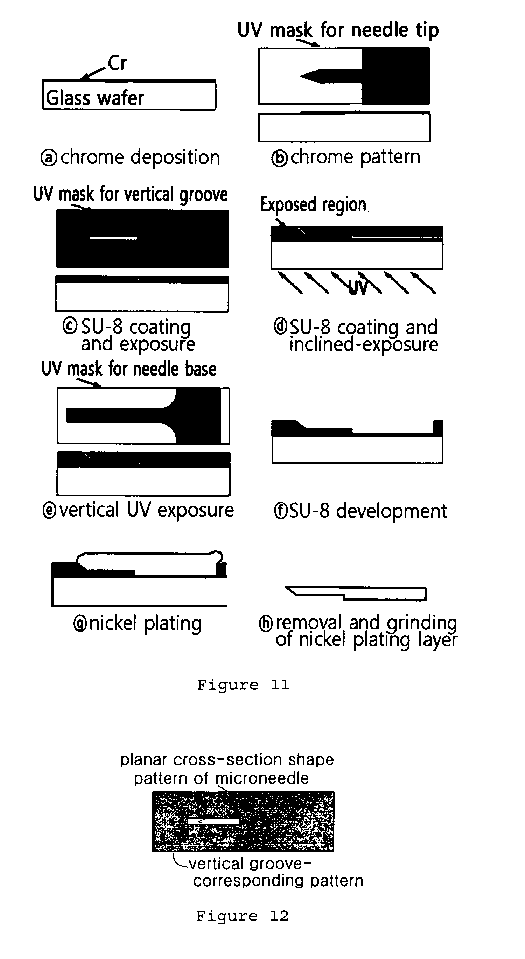

FIG. 11 is a transverse cross-sectional view illustrating another example of a process for manufacturing the microneedle formed with a vertical groove 1 according to the present invention.

In the example of the microneedle manufacturing process of FIG. 11, the light-shielding pattern is divided into a pattern corresponding to the tip 1 of the microneedle and a pattern corresponding to the base 12 of the microneedle. In other words, in the step (A), after the shape of the microneedle tip 11 is first established by using the light-shielding pattern having the shape of a planar cross-section of the tip 11 of the microneedle, the photoresist is exposed to light from the bottom surface of the light-transmitting substrate and a light-shielding pattern having the shape of a planar cross-section of the base 12 of the microneedle is placed on the top surface in the step (C). Thereafter, the photoresist is exposed to light and developed from the top surface of the light-transmitting substrate to thereby obtain the mold for microneedle formed with the vertical groove 1. The subsequent steps are the same.

**<Embodiments>**

In the following Embodiments, an ultraviolet ray was used as a light source for use in the exposure of the photoresist, a glass substrate was used as the light-transmitting substrate, a negative UV photoresist for was used as the photoresist, a titanium layer was used as the electroplating seed layer, and a nickel plating process was used as the plating process, respectively. But it is natural that the same result can be obtained through the selection of diverse methods using a variety of materials. Thus, the description of various modifications will be omitted hereinafter. In addition, an in-plane microneedle array and an out-of-plane microneedle array can also be easily manufactured based on the microneedle manufactured according to the present invention with reference to the prior art, the description thereof will be omitted. Materials and methods necessary to implement the present invention will be able to refer to prior arts including Korean Patent No. 10-0682534 which was filed by the present inventors and was registered.

Embodiment 1: Microneedle manufacturing process adopting the light-shielding pattern divided into a pattern corresponding to the tip of the microneedle and a pattern corresponding to the base of the microneedle (see FIG. 11)

UV-shielding chrome (Cr) is deposited on a glass substrate through which a ultraviolet ray can transmit, and the chrome-deposited layer is subjected to a photolithography process which employs a UV mask for a microneedle tip pattern defining the shape of the tip of the microneedle to thereby form the shape of a pointed tip of the microneedle on the chrome-deposited layer. (steps ⓐ and ⓑ).

Subsequently, SU-8 as a negative photoresist is coated to a desired depth of the vertical groove on the substrate, and then is exposed and heat-treated using a UV mask for a vertical groove pattern defining the shape of the vertical groove of the microneedle. Then, the photoresist region exposed to the UV light is cured and is not removed upon the development in a post-process to thereby form the projections for the vertical groove (step ©).

Thereafter, SU-8 is coated to a thickness larger than a desired thickness of the microneedle on the substrate, and then is inclinedly exposed to the UV light from the bottom of the glass substrate. When the UV inclined-exposure process is performed, the pattern shape of the chrome-deposited layer serves as a mask so that a three-dimensional microneedle tip shape can be defined (step ⓓ).

Next, a UV mask for a microneedle base pattern defining the shape of the base of the microneedle is placed on the substrate coated with the US-8 photoresist, which is in turn exposed and heat-treated, followed by development. Then, the US-8 photoresist region not exposed to the UV light is removed to thereby obtain a negative mold having the shape of a microneedle (steps ⓔ and ⓕ).

An electroplating seed layer is deposited on the mold and than is subjected to a nickel plating process (step ⓖ). Then, a nickel plating layer is removed from the substrate and an excessively thick plated portion is eliminated using a grinding process to thereby fabricate a microneedle having a fluid passageway formed along a side thereof (step ⓗ).

Embodiment 2: Microneedle manufacturing process adopting a pattern corresponding to the entire shape of the microneedle (see FIG. 11)

In Embodiment 2, the microneedle was manufactured in the same process as that in Embodiment 1 except the use of the UV mask for the patterns defining the entire shape including the tip and the base of the microneedle in the step ⓐ and ⓑ and the omission of the steps ⓔ and ⓕ. In the step ⓓ, preferably, the tip side of the microneedle is subjected to the inclined-exposure and the base side of the micneedle is subjected to the vertical exposure according to circumstances.

Embodiment 3: Microneedle manufacturing process adopting a different positional relationship between the tip pattern and the vertical groove pattern of the microneedle

(1) The microneedle was fabricated in the same manner as that in the Embodiment 1 or 2 by allowing the vertical groove pattern to extend beyond the tip pattern (see FIGs. 9 and 12).

As a result, as can be seen from the photograph, since an unnecessary nickel film is formed at the tip of the microneedle, a work for finishing this was required.

It was found as shown in FIG. 12 that this phenomenon occurs due to the fact that a mask pattern (indicated by doted line) defining the shape of the tip of the microneedle and a mask pattern (indicated by solid line) defining the shape of the vertical groove of the microneedle cross each other. In other words, when a photoresist is coated to a thickness corresponding to a depth of the vertical groove on the light-transmitting substrate and is exposed to light, followed by the heat treatment, the exposed portion of the photoresist layer is cured, which results in a difference in refractive index between the cured portion and the non-cured portion. In the subsequent step ⓓ, when the UV light is slantly incident to the SU-8 photoresist from the bottom of the glass substrate to define the shape of the microneedle tip, the path of light is changed at the interface between the cured portion and the non-cured portion, so that since an extended portion of the interface is not exposed to the UV light, the photoresist of this light-unexposed portion is not cured accordingly and is dissolved in a developing solution. Then, nickel is plated thereon to thereby form a nickel film.

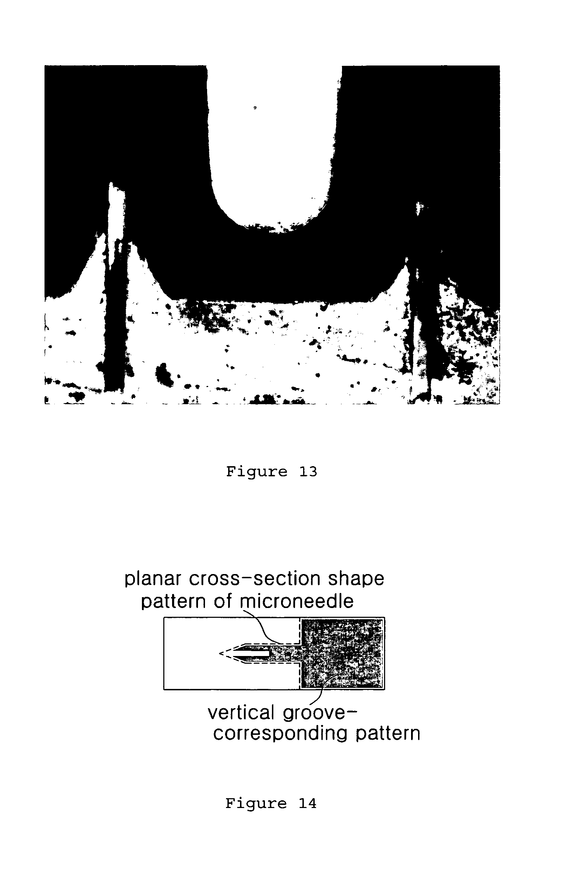

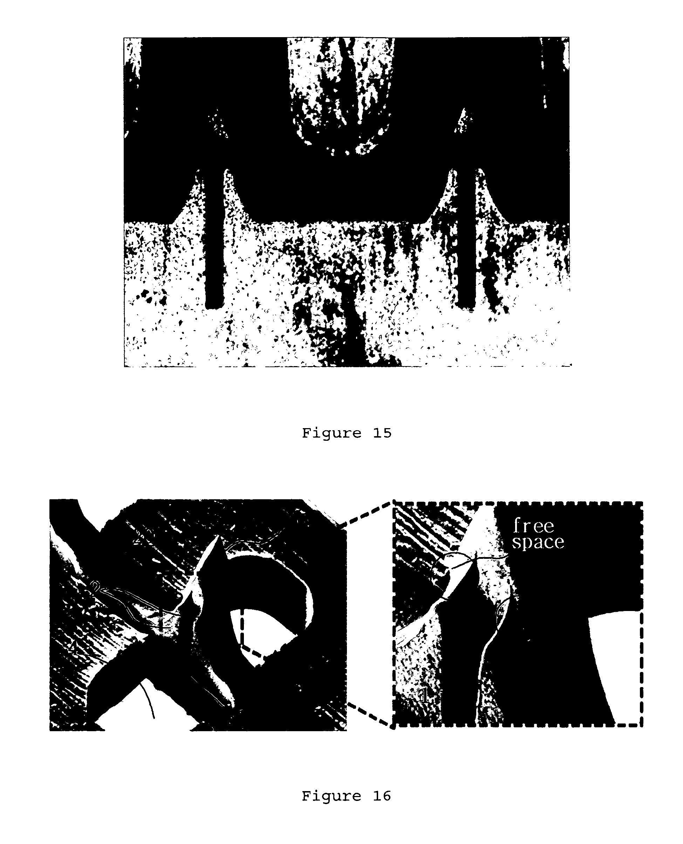

(2) In order to prevent such an unnecessary nickel film, the mask pattern (indicated by solid line) for the vertical groove of the microneedle is positioned within the mask pattern (indicated by doted line) for the microneedle tip (see FIGs. 10 and 14) to thereby fabricate the microneedle in the same method as in Embodiment 1 or 2 (see FIG. 15).

By adopting this method, as can be seen from the photograph, an unnecessary nickel film is no longer formed, so that the microneedle formed with the vertical groove for a fluid passageway could be fabricated which eliminates the necessity of a subsequent finishing work

Embodiment 4: Manufacture of out-of-plane microneedle sheet

An out-of-plane microneedle sheet 20 was manufactured which adopts the microneedle fabricated in the above-mentioned Embodiments and includes a through-recess 4 formed therein so as to store or transport a drug, the through-recess being adapted to fluidically communicate with the vertical groove 1 of the microneedle. Since the concrete manufacturing method of the out-of-plane microneedle sheet 20 is widely known in the prior art, the detailed description thereof will be omitted to avoid redundancy.

As a result, the out-of-plane microneedle sheet 20 shown in FIG. 16 could be obtained.

As described above, according to the present invention, the microneedle formed with a micro-vertical groove, which can easily transcutaneously deliver a drug without a pain, can be manufactured through the exposure, development and plating processes only. In addition, since the microneedle is fabricated in a state where the tip thereof has been finished, a process of finishing the manufactured product can also be omitted. Thus, an economical and high-quality microneedle can be provided.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

## Claims

1. A microneedle which is configured to have a vertical groove formed along a side thereof so as to serve as a fluid passageway and in which an upper portion of an outer edge of each of a pair of projections forming both sidewalls of the vertical groove is formed to be narrower than an outer edge of the body defining a bottom of the vertical groove and to be shorter than an upper portion of the body.

2. The microneedle according to claim 1, wherein the pair of projections and the body become gradually narrower as they go toward the top.

3. A method for manufacturing the microneedle according to claim 1 or 2, comprising the steps of:
(a) forming a light-shielding pattern having the shape of a planar cross-section of the microneedle on a light-transmitting substrate;
(b) coating the light-transmitting substrate with a photoresist, masking the photoresist in a light-shielding pattern corresponding to the vertical groove, and exposing the masked photoresist to light to thereby define projections for the vertical groove;
(c) coating the light-transmitting substrate with a photoresist, exposing and developing the photoresist from the bottom surface of the light-transmitting substrate to thereby form a mold for the microneedle formed with the vertical groove;
(d) depositing an electroplating seed layer on the top surface of the mold; and
(e) removing a plating layer formed by performing a plating process on the electroplating seed layer.

4. The method according to claim 3, wherein the light-shielding pattern corresponding to the vertical groove in the step (b) is configured in such a fashion that a slit corresponding to the vertical groove is formed in a tip of the microneedle, and the width and length thereof are smaller by a predetermined dimension than those of the light-shielding pattern having the planar cross-section shape of the microneedle in the step (a).

5. The method according to claim 4, wherein the light-shielding pattern having the planar cross-section shape of the microneedle and the light-shielding pattern corresponding to the vertical groove become narrower as they go toward the top.

6. The method according to claim 3, wherein in the step (a), the light-shielding pattern has the shape of a planar cross-section of a tip of the microneedle, and the step (c) comprises exposing the photoresist to light from the bottom surface of the light-transmitting substrate, placing a light-shielding pattern having the shape of a planar cross-section of a base of the microneedle on the top surface of the light-transmitting substrate, and exposing and developing the photoresist from the top surface of the light-transmitting substrate.

7. The method according to claim 3, wherein the light-shielding pattern in the step (a) has the shape of the planar cross-section of one or more microneedles which are arranged in parallel with each other.

8. The method according to claim 3, wherein the exposing of the photoresist in the step (c) is performed in an inclined manner.

9. The method according to claim 3, further comprising the steps of:
(f) fabricating a negative mold using the removed plating layer as an original form; and
(e) obtaining a molded product made of a polymer material using the negative mold.

10. An out-of-plane microneedle sheet to which the microneedle according to claim 1 or 2 is applied.

11. The out-of-plane microneedle sheet according to claim 10, wherein the sheet comprises a through-recess formed therein so as to store or transport a drug, the through-recess being adapted to fluidically communicate with the vertical groove of the microneedle.
